# EUROPEAN PATENT APPLICATION

(11) **EP 4 817 649 A1**
(43) Date of publication of application: **07.10.2026**
(21) Application number: 25745159.1
(22) Date of filing: 22.01.2025
(51) Int. Cl.: A61M 1/16

(54) **HEMODIALYSIS SYSTEM**

(30) Priority: 24.01.2024 JP 2024008867
(71) Applicant: Nihon Trim Co., Ltd., Osaka 531-0076 (JP)
(72) Inventor: MIYAKE, Masato, Osaka-shi, Osaka 531-0076 (JP)
(74) Representative: Manitz Finsterwald Patent- und Rechtsanwaltspartnerschaft mbB
(86) International application number: PCT/JP2025/001884
(87) International publication number: WO 2025/159118

(57) **Abstract**

A hemodialysis system 100 comprises a hydrogen adding device 1 that adds hydrogen to water, a dialyzing water producing device 2 that produces dialyzing water, a dialysis fluid preparing device 3 that adds a medicinal substance to the dialyzing water to prepare dialysis fluid, a concentration adjusting device 4 that adjusts the dissolved hydrogen concentration of the dialysis fluid, and a dialysis machine 5 that filters patient's blood using the dialysis fluid.

## Description

### Technical Field

The present invention relates to a hemodialysis system.

### Background Technology

In recent years, dialysis treatment using dialysis fluid with hydrogen gas dissolved therein has been attracting attention.
For example, it has been known that electrolyzed hydrogen water produced by electrolyzing water contributes to reducing patient's oxidative stress (see, for example, Patent Document 1 below).

### Prior art documents

### Patent documents

Patent Document 1: Japanese Patent Application Publication No. 2015-139475

### Summary of the Invention

### Problems to be solved by the Invention

In a hemodialysis system, dialysis fluid with hydrogen gas dissolved therein is produced by using a hydrogen adding device for adding hydrogen to water. For such system, it is desirable to be operated with a dialysis fluid having a desired dissolved hydrogen concentration.

However, if there is a waiting period after hydrogen addition before treatment begins, the concentration of dissolved hydrogen in the dialysis fluid gradually decreases. Further, the dissolved hydrogen concentration of the dialysis fluid gradually decreases as the dialysis fluid travels along the path from the hydrogen adding device to the dialysis machine.

Therefore, in dialysis treatment for multiple patients using multiple dialysis machines, the routes for supplying dialysis fluid to the respective dialysis machines are different, and the dissolved hydrogen concentration of the dialysis fluid may vary from dialysis machine to dialysis machine. If the dissolved hydrogen concentration of the dialysis fluid is varied, it becomes difficult to provide treatment tailored to the patient's constitution and symptoms.

In view of the above actual situations, the present invention was made, and a primary objective thereof is to provide a hemodialysis system which can suppress the variation of the dissolved hydrogen concentration of the dialysis fluid.

### Means for solving the problem

A hemodialysis system according to the present invention comprises:
a dialyzing water producing device that purifies supplied water and produces dialyzing water;
a dialysis fluid preparing device that adds a medicinal substance to the dialyzing water to prepare a dialysis fluid to be used in hemodialysis treatment;
a dialysis machine that filters patient's blood using the dialysis fluid;
a hydrogen adding device that adds hydrogen to at least one of the water supplied to the dialyzing water producing device, the dialyzing water produced by the dialyzing water producing device, and the dialysis fluid prepared by the dialysis fluid preparing device; and
a concentration adjusting device for adjusting the dissolved hydrogen concentration of the dialysis fluid to be used by the dialysis machine.

### Effect of the Invention

The hemodialysis system according to the present invention has the above-described configuration, therefore, it becomes possible to suppress variations of the dissolved hydrogen concentration of the dialysis fluid so as to maintain an arbitrary dissolved hydrogen concentration.

### Brief description of the Drawings

[FIG. 1] A block diagram showing the schematic configuration of a hemodialysis system according to the present invention.
[FIG. 2] A diagram showing the detailed configuration of the hydrogen adding device of FIG. 1.
[FIG. 3] A block diagram showing a modification of the hemodialysis system of FIG. 1.
[FIG. 4] A block diagram showing another modification of the hemodialysis system of FIG. 1.
[FIG. 5] A block diagram showing yet another modification of the hemodialysis system of FIG. 1.
[FIG. 6] A block diagram showing a modification of the hemodialysis system of FIG. 5.
[FIG. 7] A block diagram showing a modification of the hemodialysis system of FIG. 6.
[FIG. 8] A block diagram showing a modification of the hemodialysis system of FIG. 6.

### Mode for carrying out the Invention

An embodiment of the present invention will now be described with reference to the drawings.

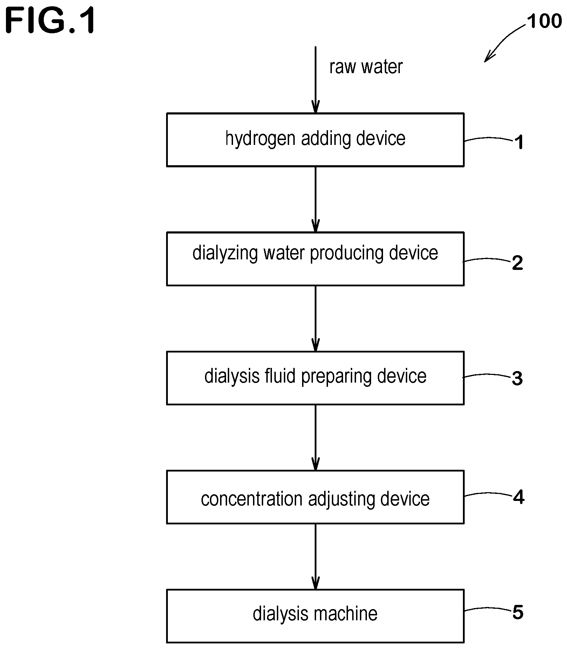

FIG. 1 shows the flow path configuration of a hemodialysis system 100 in the present embodiment.
The hemodialysis system 100 in the present embodiment comprises a hydrogen adding device 1, a dialyzing water producing device 2, a dialysis fluid preparing device 3, a concentration adjusting device 4, and a dialysis machine 5.

The hydrogen adding device 1 adds hydrogen to water supplied to the dialyzing water producing device 2. The hydrogen adding device 1 of the present embodiment is arranged upstream of the dialyzing water producing device 2. The hydrogen adding device 1 is supplied with raw water.

As the raw water, tap water is generally used, but others such as well water, groundwater and the like can be used.
The raw water is subjected to pretreatment such as softening and then supplied to the hydrogen adding device 1.
The raw water to which hydrogen has been added by the hydrogen adding device 1 is, as dissolved hydrogen water in which hydrogen has been dissolved, supplied to the dialyzing water producing device 2.

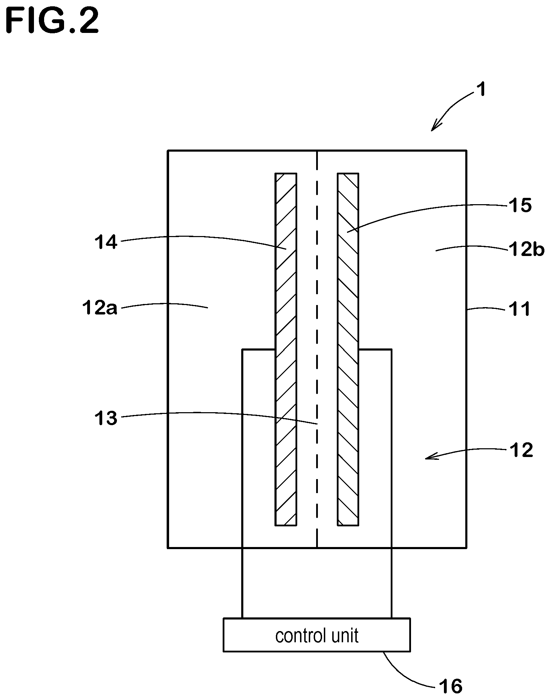

FIG. 2 shows the details of the hydrogen adding device 1 of the present embodiment. The hydrogen adding device 1 comprises an electrolytic cell 11 that generates hydrogen gas by electrolyzing the supplied raw water, and a control unit 16 that controls the electrolytic cell 11.

The electrolytic cell 11 has an electrolysis chamber 12 in which electrolysis is carried out, a diaphragm 13, an anode electrode 14, and a cathode electrode 15.

The electrolysis chamber 12 is divided by the diaphragm 13 into an anode chamber 12a on the anode electrode 14 side and a cathode chamber 12b on the cathode electrode 15 side. The raw water is supplied to the anode chamber 12a and the cathode chamber 12b, for example, via a bifurcated flow path (not shown).

The diaphragm 13 is, for example, a solid polymer membrane.
The anode electrode 14 is disposed on one side of the diaphragm 13, and the cathode electrode 15 is disposed on the other side of the diaphragm.

The anode electrode 14 and the cathode electrode 15 are supplied with a direct current. The direct current supplied to the anode electrode 14 and the cathode electrode 15 is controlled by the control unit 16.

The control unit 16 controls each part of the hydrogen adding device 1.
The control unit 16 includes, for example, a CPU (Central Processing Unit) that executes various types of arithmetic processing, information processing, etc., and a memory that stores programs that control the operations of the CPU and various types of information. The various functions of the control unit 16 are realized by the CPU, the memory, and the program.

The control unit 16 feedback controls the electrolytic voltage applied to the anode electrode 14 and the cathode electrode 15 so that the electrolytic current supplied to the anode electrode 14 and the cathode electrode 15 becomes a predetermined desired value. For example, if the electrolysis current is too high, the control unit 16 reduces the voltage, and if the electrolysis current is too low, the control unit 16 increases the voltage.
Thereby, the electrolysis current is appropriately controlled.
The electrolysis current is measured by a measuring unit, for example, provided in a circuit that supplies power to the anode electrode 14 and the cathode electrode 15.

Of the electrolyzed water electrolyzed in the electrolytic cell 11, the electrolyzed water produced in the cathode chamber 12b is supplied to the dialyzing water producing device 2 as cathode water. The other electrolyzed water produced in the anode chamber 12a is discharged to the outside of the hydrogen adding device 1 as anode water.

By electrolyzing the water in the electrolysis chamber 12, oxygen gas is generated in the anode chamber 12a and hydrogen gas is generated in the cathode chamber 12b.

The oxygen gas generated in the anode chamber 12a dissolves in the electrolyzed water in the anode chamber 12a, is taken out of the anode chamber 12a as anode water, and is discharged to the outside of the hydrogen adding device 1.

The hydrogen gas generated in the cathode chamber 12b dissolves in the electrolyzed water in the cathode chamber 12b, is taken out of the cathode chamber 12b as cathode water, and is supplied to the dialyzing water producing device 2.
That is, the cathode water which is sent from the cathode chamber 12b to the dialyzing water producing device 2 is electrolyzed hydrogen water which is electrolyzed in the cathode chamber 12b and in which hydrogen gas is dissolved.

In addition to the form including the electrolytic cell 11 as described above, there may be applied to the hydrogen adding device 1,
a device in a form in which hydrogen gas generated by, for example, chemically reacting water with magnesium is dissolved in water, or
a device in a form in which hydrogen gas pressurized in a cylinder or the like is dissolved in water.

The hydrogen adding device 1 or the downstream side thereof may be provided with a tank for storing the produced electrolyzed hydrogen water.
Such configuration makes it possible to supply a large amount of electrolyzed hydrogen water to the dialyzing water producing devices 2 at one time, which can be easily adapted to dialysis treatment for multiple patients.

The dialyzing water producing device 2 is arranged between the hydrogen adding device 1 and the dialysis fluid preparing device 3.

The dialyzing water producing device 2 purifies the water supplied from the hydrogen adding device 1 and produces dialyzing water. A form for realizing such a configuration includes a reverse osmosis treatment device, for example.

The reverse osmosis treatment device filters the water supplied from the hydrogen adding device 1 using a reverse osmosis membrane.
The reverse osmosis treatment device comprises
a reverse osmosis membrane module having the reverse osmosis membrane, and
a pump for pumping the water supplied from the hydrogen adding device 1 to the reverse osmosis membrane module.

In the hemodialysis system 100, the dialyzing water producing device 2 may be a form of filter other than the reverse osmosis treatment that satisfies the standards for dialyzing water, for example, one utilizing an ion exchange (EDI: Electro-deionization) module.

Since hydrogen is dissolved in the water supplied from the hydrogen adding device 1, hydrogen is also dissolved in the dialyzing water produced by the dialyzing water producing device 2. That is, the dialyzing water producing device 2 produces hydrogen water for dialysis.
The hydrogen water for dialysis produced by the dialyzing water producing device 2 is supplied to the dialysis fluid preparing device 3.

The dialyzing water producing device 2 or the downstream side thereof may be provided with a tank for storing the produced hydrogen water for dialysis.
Such configuration makes it possible to supply a large amount of hydrogen water for dialysis to the dialysis fluid preparing device 3 at one time, which can be easily adapted to dialysis treatment for multiple patients.
The dialyzing water producing device 2 may be provided with a pressure reducing valve for degassing hydrogen gas contained in the dialyzing water.

The dialysis fluid preparing device 3 is arranged between the dialyzing water producing device 2 and the concentration adjusting device 4.
The dialysis fluid preparing device 3 mixes a medicinal substance with the hydrogen water for dialysis produced by the dialyzing water producing device 2, and prepares the dialysis fluid to be used in hemodialysis treatment.
As the medicinal substance to be added to the hydrogen water for dialysis, dialysis fluid concentrate as well as powder of dialysate concentrate may be used.

Since hydrogen is dissolved in the hydrogen water for dialysis supplied from the dialyzing water producing device 2, hydrogen is also dissolved in the dialysis fluid prepared by the dialysis fluid preparing device 3.

The dialysis fluid preparing device 3 or the downstream side thereof may be provided with a tank for storing the produced dialysis fluid. Such configuration makes it possible to supply a large amount of the hydrogen water for dialysis to the dialysis machine 5 at one time, which can be easily adapted to dialysis treatment for multiple patients.
The dialysis fluid preparing device 3 may be provided with a pressure reducing valve for degassing hydrogen gas contained in the dialysis fluid.

The concentration adjusting device 4 is arranged between the dialysis fluid preparing device 3 and the dialysis machine 5.
The concentration adjusting device 4 may be arranged at the dialysis fluid preparing device 3 or a tank provided downstream thereof. Further, the concentration adjusting device 4 may be disposed inside the dialysis machine 5.
The concentration adjusting device 4 adjusts the dissolved hydrogen concentration of the dialysis fluid prepared by the dialysis fluid preparing device 3.

The concentration adjusting device 4 may be arranged between the dialyzing water producing device 2 and the dialysis fluid preparing device 3.
In this case, the concentration adjusting device 4 adjusts the dissolved hydrogen concentration of the dialyzing water to be supplied to the dialysis fluid preparing device 3, thereby adjusting the dissolved hydrogen concentration of the dialysis fluid.

The concentration adjusting device 4 includes
a device for increasing the dissolved hydrogen concentration of the dialysis fluid and
a device for decreasing the dissolved hydrogen concentration of the dialysis fluid.

The device for increasing the dissolved hydrogen concentration of the dialysis fluid includes a device having the same configuration as the hydrogen adding device 1. Namely, in addition to the mode including the electrolytic cell 11 shown in FIG. 2, there may be applied to the concentration adjusting device 4, for example,
a device of a mode in which hydrogen gas generated by chemically reacting water with magnesium is made dissolved into the dialysis fluid, or
a device of a mode in which hydrogen gas pressurized inside a cylinder or the like is made dissolved into the dialysis fluid.
In this case, the electrolytic cell 11 and the like constituting the hydrogen adding device 1 may be configured to also serve as at least a part of the concentration adjusting device 4.

The device for decreasing the dissolved hydrogen concentration in dialysis fluid includes those of
a mode in which the hydrogen gas contained in the dialysis fluid is made degassed by opening and closing a pressure reducing valve, and
a mode in which the dialysis fluid containing dissolved hydrogen is made mixed and diluted with dialysis fluid not containing dissolved hydrogen.
In the former case, a pressure reducing valve provided in at least one of the dialysis fluid preparing device 3 and the dialysis machine 5 may be configured to also serve as at least a part of the concentration adjusting device 4.
Further, in the mode in which at least one of the dialyzing water producing device 2 and the dialysis fluid preparing device 3 is provided with a pressure reducing valve for degassing hydrogen gas contained in the dialyzing water,
this pressure reducing valve may be configured to also serve as at least a part of the concentration adjusting device 4.

The dialysis fluid whose dissolved hydrogen concentration has been adjusted by the concentration adjusting device 4 is supplied to the dialysis machine 5.
That is, the concentration adjusting device 4 adjusts the dissolved hydrogen concentration of the dialysis fluid to be used by the dialysis machine 5.
Thereby, the dissolved hydrogen concentration of the dialysis fluid is stabilized, and the dialysis fluid having the desired dissolved hydrogen concentration is supplied to the dialysis machine 5.

The dialysis machine 5 filters patient's blood using the dialysis fluid supplied from the concentration adjusting device 4. This makes it possible to carry out dialysis treatment using the dialysis fluid in which hydrogen has been dissolved.
The dialysis machine 5 is composed of, for example, a dialyzer (artificial kidney).

The blood taken from the patient's body is pumped into the dialyzer.
The dialyzer has a hollow-fiber membrane that separates the blood from the dialysis fluid. The hollow-fiber membrane transfers waste products contained in the blood to the dialysis fluid, and purifies the blood. The blood purified by the dialyzer is returned to the patient's body. That is, the dialysis machine 5 purifies the blood while circulating the blood between the patient's body and the dialyzer.
The dialysis machine 5 may be provided with a pressure reducing valve for degassing hydrogen gas contained in the dialysis fluid.

In the hemodialysis system 100, as the concentration adjusting device 4 adjusts the dissolved hydrogen concentration of the dialysis fluid, fluctuations in the dissolved hydrogen concentration of the dialysis fluid are suppressed, and it becomes possible to maintain an arbitrary dissolved hydrogen concentration. And, it becomes possible to perform dialysis treatment using the dialysis fluid with a dissolved hydrogen concentration that is appropriate for the patient's constitution and symptoms.

In the hemodialysis system according to the present invention, it is sufficient that the hydrogen adding device 1 is configured to add hydrogen to at least one of the water supplied to the dialyzing water producing device 2, the dialyzing water produced by the dialyzing water producing device 2, and the dialysis fluid prepared by the dialysis fluid preparing device 3.
Thus, the hemodialysis system 100 may be modified as shown below, for example.

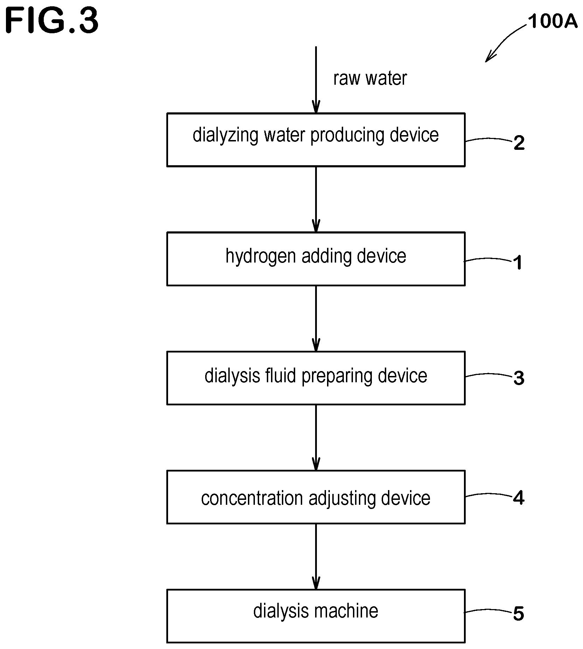

FIG. 3 is a block diagram of a hemodialysis system 100A which is a modified example of the hemodialysis system 100 of FIG. 1. For the parts of the hemodialysis system 100A that are not described below, the above described configurations in the hemodialysis system 100 can be applied.

In the hemodialysis system 100A, the hydrogen adding device 1 is arranged between the dialyzing water producing device 2 and the dialysis fluid preparing device 3.

The dialyzing water producing device 2 purifies the supplied raw water and produces dialyzing water. The dialyzing water produced by the dialyzing water producing device 2 is supplied to the hydrogen adding device 1, and hydrogen is added.
Thereby, hydrogen water for dialysis which is the dialyzing water in which hydrogen has been dissolved is produced.

Thereafter, similarly to the hemodialysis system 100, the dialysis fluid containing dissolved hydrogen is prepared by the dialysis fluid preparing device 3, the concentration of the dissolved hydrogen is adjusted by the concentration adjusting device 4, and the dialysis fluid is supplied to the dialysis machine 5.
Thereby, it becomes possible to suppress fluctuations in the dissolved hydrogen concentration of the dialysis fluid. And, it becomes possible to perform dialysis treatment using the dialysis fluid with a dissolved hydrogen concentration which is appropriate for the patient's constitution and symptoms.

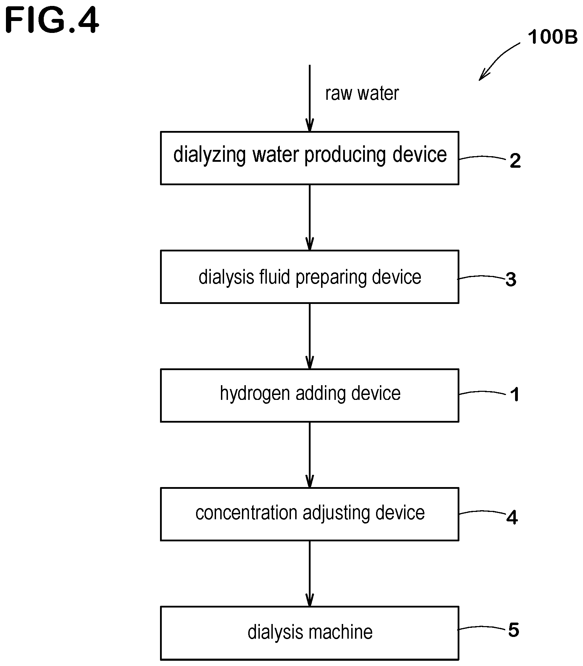

FIG. 4 is a block diagram of a hemodialysis system 100B, which is another modified example of the hemodialysis system 100 of FIG. 1. For the parts of the hemodialysis system 100B that are not described below, the above described configurations in the hemodialysis systems 100 and 100A can be applied.

In the hemodialysis system 100B, the hydrogen adding device 1 is arranged between the dialysis fluid preparing device 3 and the concentration adjusting device 4.

The dialyzing water producing device 2 purifies the supplied raw water and produces dialyzing water. The dialyzing water produced by the dialyzing water producing device 2 is supplied to the dialysis fluid preparing device 3, and the dialysis fluid is prepared. The dialysis fluid prepared by the dialysis fluid preparing device 3 is supplied to the hydrogen adding device 1, and hydrogen is added. Thereby, a dialysis fluid in which hydrogen has been dissolved is produced.

Thereafter, similarly to the hemodialysis system 100 and the like, the dissolved hydrogen concentration is adjusted by the concentration adjusting device 4 and supplied to the dialysis machine 5. Thereby, it becomes possible to suppress fluctuations in the dissolved hydrogen concentration of the dialysis fluid. And, it becomes possible to perform dialysis treatment using the dialysis fluid with a dissolved hydrogen concentration that is appropriate for the patient's constitution and symptoms.

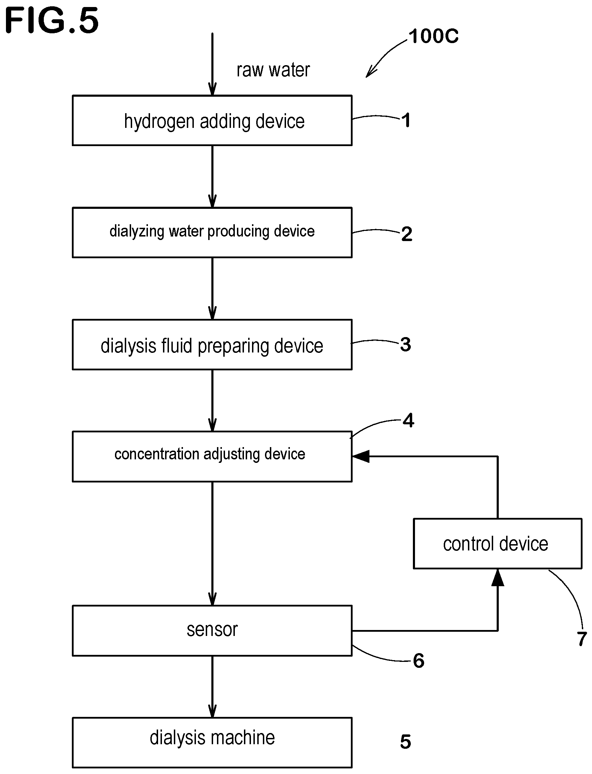

FIG. 5 is a block diagram of a hemodialysis system 100C, which is yet another modified example of the hemodialysis system 100 of FIG. 1. For the parts of the hemodialysis system 100C that are not described below, the above described configurations in the hemodialysis systems 100, 100A and 100B can be applied.

The hemodialysis system 100C differs from the hemodialysis system 100 etc. in that it includes a sensor 6 that measures the dissolved hydrogen concentration of the dialysis fluid to be used by the dialysis machine 5, and a control device 7 that controls the concentration adjusting device 4.

The sensor 6 is disposed between the concentration adjusting device 4 and the dialysis machine 5. The sensor 6 may be disposed inside the concentration adjusting device 4 or inside the dialysis machine 5.
The sensor 6 measures the dissolved hydrogen concentration of the dialysis fluid supplied to the dialysis machine 5, and outputs an electrical signal corresponding to the measured dissolved hydrogen concentration to the control device.

The control device 7 comprises, for example, a CPU (Central Processing Unit) that executes various types of arithmetic processing, information processing, etc., and a memory that stores programs that control the operation of the CPU and various types of information. The various functions of the control device 7 are realized by the CPU, memory and program.

To the control device 7 and the control unit 16, a CPU or the like in which the functions of both are integrated may be applied.

The control device 7 controls the concentration adjusting device 4 based on the electrical signals output from the sensor 6.
When the dissolved hydrogen concentration of the dialysis fluid is lower than the target value, the control device 7 controls the concentration adjusting device 4 so as to increase the dissolved hydrogen concentration.
When the dissolved hydrogen concentration of the dialysis fluid is higher than the target value, the control device 7 controls the concentration adjusting device 4 so as to lower the dissolved hydrogen concentration.
Thereby, it becomes possible to easily suppress fluctuations in the dissolved hydrogen concentration of the dialysis fluid. And, it becomes possible to easily perform dialysis treatment using the dialysis fluid with a dissolved hydrogen concentration that is appropriate for the patient's constitution and symptoms.

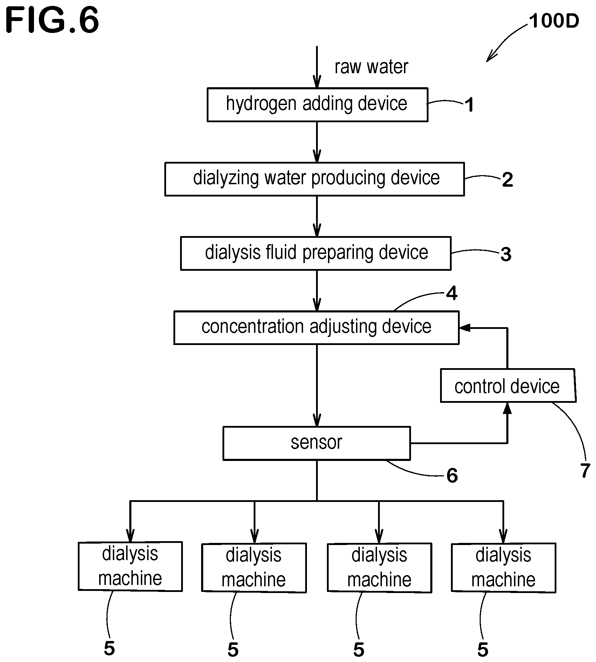

FIG. 6 is a block diagram of a hemodialysis system 100D which is a modified example of the hemodialysis system 100C of FIG. 5. For the parts of the hemodialysis system 100D that are not described below, the above described configurations in the hemodialysis systems 100C can be applied.

The hemodialysis system 100D differs from the hemodialysis system 100C in that it includes a plurality of dialysis machines 5.
According to the hemodialysis system 100D, dialysis treatment for multiple patients can be performed, simultaneously, using the multiple dialysis machines 5.

In the hemodialysis system 100D, in order to be able to supply the dialysis fluid to the multiple dialysis machines 5, it is desirable to provide a tank for storing the dialyzing water, for example, between the dialyzing water producing device 2 and the dialysis fluid preparing device 3. The tank may be provided between the hydrogen adding device 1 and the dialyzing water producing device 2 or between the dialysis fluid preparing device 3 and the concentration adjusting device 4.

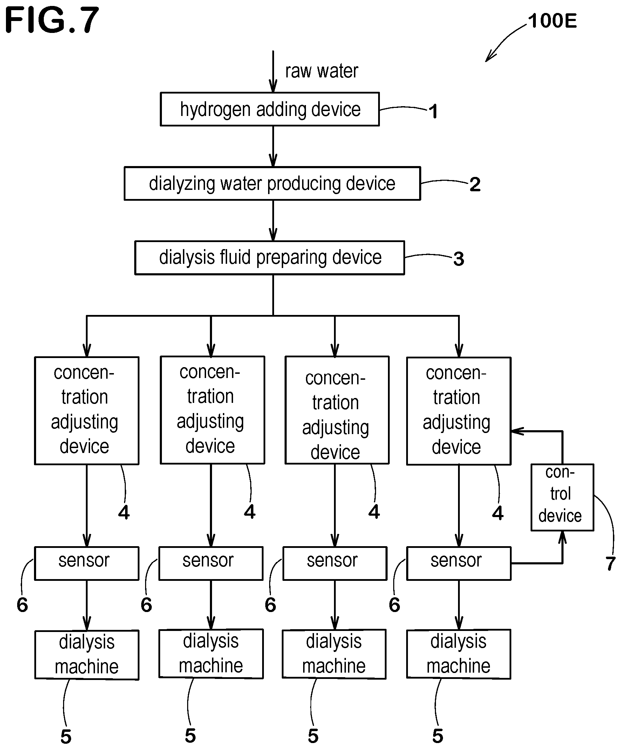

FIG. 7 is a block diagram of a hemodialysis system 100E which is a modified example of the hemodialysis system 100D of FIG. 6. For the parts of the hemodialysis system 100E that are not described below, the above described configurations in the hemodialysis system 100D can be applied.

The hemodialysis system 100E differs from the hemodialysis system 100D in that it includes the concentration adjusting device 4 and the sensor 6 for each of the dialysis machines 5. That is, the hemodialysis system 100E includes a plurality of the dialysis machines 5, a plurality of the concentration adjusting devices 4, and a plurality of the sensors 6.

Each concentration adjusting device 4 adjusts the dissolved hydrogen concentration of the dialysis fluid supplied to the associated dialysis machine 5.
Each sensor 6 measures the dissolved hydrogen concentration of the dialysis fluid supplied to the associated dialysis machine 5. The measurement results of the respective sensors 6 are, as electrical signals, output to the control device 7.

Based on the electrical signals output from the respective sensors 6, the control device 7 individually controls the corresponding concentration adjusting devices 4.
That is, the control device 7 controls the corresponding concentration adjusting device 4 so that the dissolved hydrogen concentration becomes a predetermined value set for each dialysis machine 5. The control device 7 may be provided for each of the sensors 6.

In the hemodialysis system 100E, the dissolved hydrogen concentrations of the dialysis fluids supplied to the respective dialysis machines 5 may differ for each dialysis machine 5 or may be the same.

When the dissolved hydrogen concentrations of the dialysis fluids supplied to the respective dialysis machines 5 are controlled to be the same,
the dissolved hydrogen concentrations of all the dialysis fluids supplied simultaneously to the multiple dialysis machines 5 are made uniform, and
it becomes possible to easily suppress fluctuations in the dissolved hydrogen concentration of the dialysis fluid for each dialysis machine 5.

When the dissolved hydrogen concentrations of the dialysis fluids supplied to the respective dialysis machines 5 are controlled to be different for each dialysis machine 5, it becomes possible to easily perform dialysis treatment using the dialysis fluids with an individual dissolved hydrogen concentration according to the constitution and symptoms of each patient, even when dialysis treatments are performed on multiple patients at the same time.

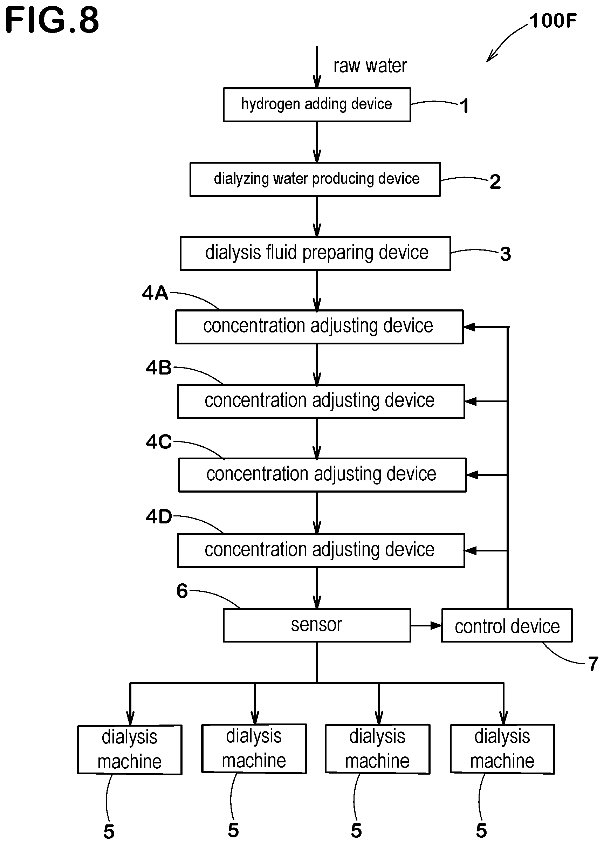

FIG. 8 is a block diagram of a hemodialysis system 100F which is another modified example of the hemodialysis system 100D of FIG. 6. For the parts of the hemodialysis system 100F that are not described below, the above described configurations in the hemodialysis system 100D can be applied.

The hemodialysis system 100F differs from the hemodialysis system 100D in that it includes a plurality of different types of concentration adjusting devices 4A to 4D. The concentration adjusting devices 4A to 4D used in the hemodialysis system 100F include the above described device for increasing the dissolved hydrogen concentration of the dialysis fluid and device for decreasing the dissolved hydrogen concentration of the dialysis fluid.
The configurations of the concentration adjusting devices 4A to 4D are not particularly limited, but it is desirable that they include
one of the above described devices for increasing the dissolved hydrogen concentration of the dialysis fluid and
one of the above described devices for decreasing the dissolved hydrogen concentration of the dialysis fluid.

In the hemodialysis system 100F, based on the electrical signal output from the sensor 6, from among the plurality of types of concentration adjusting devices 4A to 4D, the control device 7 selects and controls a concentration adjusting device to be operated.

For example, when it is desired to rapidly increase the dissolved hydrogen concentration of the dialysis fluid, the control device 7 fully operates the concentration adjusting devices 4A and 4B having the configuration which includes the electrolytic cell 11, and the configuration in which hydrogen gas is pressurized inside a cylinder or the like.

On the other hand, when it is desired to rapidly lower the dissolved hydrogen concentration of the dialysis fluid, the control device 7 fully operates the concentration adjusting devices 4C and 4D having the configuration which degases the hydrogen gas, and the configuration which dilutes the dialysis fluid with hydrogen is dissolved therein.

The concentration adjusting devices 4A to 4D having the different configurations do not necessarily need to be arranged in a concentrated manner as shown in FIG. 8, but may be individually and dispersedly installed, for example, between the hydrogen adding device 1 and the dialyzing water producing device 2, and between the dialyzing water producing device 2 and the dialysis fluid preparing device 3.
On the other hand, in the configurations of the hemodialysis systems 100A to 100E, the concentration adjusting device 4 may be replaced with the concentration adjusting devices 4A to 4D having different configurations.
Here, each of the concentration adjusting devices 4A to 4D may be installed anywhere between the hydrogen adding device 1 and the sensor 6.
Further, the hydrogen adding device 1 may be configured to serve as a part of the concentration adjusting device 4A to 4D, and
the pressure reducing valve provided in at least one of the dialysis machine 5, the dialysis fluid preparing device 3 and the dialyzing water producing device 2, may be configured to serve as a part of the concentration adjusting device 4A to 4D.

When it is desired to control the dissolved hydrogen concentration of the dialysis fluid with high precision, the control device 7 operates the concentration adjusting device 4A configured to include the electrolytic cell 11 that can control the amount of generated hydrogen gas by an electrolytic current.
Further, when fine adjustment of the dissolved hydrogen concentration of the dialysis fluid is desired, the control device 7 operates the concentration adjusting device 4A, 4D of the configuration including the electrolytic cell 11, the configuration diluting the dialysis fluid in which hydrogen has been dissolved, and the like.

In the hemodialysis system 100F, the control device 7 may be configured to determine priority order of the operation of each concentration adjusting device 4A to 4D based on the output from the sensor 6, and to control each concentration adjusting device 4A to 4D based on the priority order.

The priority order of the operations of the concentration adjusting devices 4A to 4D is determined using a learning model generated by deep learning in advance using artificial intelligence (AI), for example.

The learning model is, for example, defined by an intermediate layer generated by machine learning, with the output from the sensor 6 that is the dissolved hydrogen concentration of the dialysis fluid as the input layer, and the priority order of the operation of each concentration adjusting device 4 as the output layer.

While detailed descriptions have been made of the hemodialysis systems 100 and others according to the present invention, the present invention can be implemented by modifying in various modes without being limited to the above-described specific embodiments.

For example, it is desirable that the hemodialysis system 100 to 100F is provided with an operating device (not shown) for arbitrarily operating the control device 7 or the concentration adjusting device 4.
The operating device may be provided in at least one of the dialyzing water producing device 2, the dialysis fluid preparing device 3 and the dialysis machine 5 so that a doctor, nurse, etc. can remotely or closely operate the control device 7 or the concentration adjusting device 4 from near the patient, etc.
The operation device is composed of, for example, various buttons, a touch panel and the like that allow a doctor or nurse to input operations.

### [Additional statement]

The present invention includes the following embodiments.

### [Present Invention 1]

A hemodialysis system comprising:
a dialyzing water producing device that purifies supplied water and produces dialyzing water;
a dialysis fluid preparing device that adds a medicinal substance to the dialyzing water to prepare a dialysis fluid to be used in hemodialysis treatment;
a dialysis machine that filters patient's blood using the dialysis fluid;
a hydrogen adding device that adds hydrogen to at least one of the water supplied to the dialyzing water producing device, the dialyzing water produced by the dialyzing water producing device, and the dialysis fluid prepared by the dialysis fluid preparing device; and
a concentration adjusting device for adjusting the dissolved hydrogen concentration of the dialysis fluid to be used by the dialysis machine.

### [Present Invention 2]

The hemodialysis system as set forth in Present Invention 1, which comprises
a sensor for measuring the dissolved hydrogen concentration of the dialysis fluid to be used by the dialysis machine; and
a control device that controls the concentration adjusting device based on an output of the sensor.

### [Present Invention 3]

The hemodialysis system as set forth in Present Invention 2, which comprises a plurality of the dialysis machines.

### [Present Invention 4]

The hemodialysis system as set forth in Present Invention 3, which comprises the concentration adjusting device and the sensor corresponding to each of the dialysis machines.

### [Present Invention 5]

The hemodialysis system as set forth in Present Invention 4, wherein the control device controls the concentration adjusting devices so as to equalize the dissolved hydrogen concentrations of all of the dialysis fluids simultaneously supplied to the plurality of the dialysis machines.

### [Present Invention 6]

The hemodialysis system as set forth in Present Invention 4, wherein the control device controls the concentration adjusting devices so that the dialysis fluids respectively supplied to the dialysis machines have different dissolved hydrogen concentrations.

### [Present Invention 7]

The hemodialysis system as set forth in any one of Present Inventions 1 to 6, wherein the concentration adjusting device includes a plurality of types of concentration adjusting devices.

### [Present Invention 8]

The hemodialysis system as set forth in Present Invention 7, which comprises:
a sensor for measuring the dissolved hydrogen concentration of the dialysis fluid to be used by the dialysis machine; and
a control device that determines a priority order for the operation of each concentration adjusting device based on the output of the sensor and controls each concentration adjusting device based on the priority order.

### [Present Invention 9]

The hemodialysis system as set forth in any one of Present Inventions 1 to 8, wherein the hydrogen adding device includes an electrolytic cell that generates hydrogen gas by electrolyzing the supplied water.

### [Present Invention 10]

The hemodialysis system as set forth in Present Invention 9, wherein the electrolytic cell is also usable as the concentration adjusting device.

### [Present Invention 11]

The hemodialysis system as set forth in any one of Present Inventions 1 to 10, wherein at least one of the dialyzing water producing device, the dialysis fluid preparing device and the dialysis machine is provided with a pressure reducing valve for degassing hydrogen gas contained in the dialyzing water or the dialysis fluid, and the pressure reducing valve is also usable as the concentration adjusting device.

### [Present Invention 12]

The hemodialysis system as set forth in any one of Present Inventions 1 to 11, wherein at least one of the dialyzing water producing device, the dialysis fluid preparing device and the dialysis machine is provided with an operating device for arbitrarily operating the control device or the concentration adjusting device.

### Explanation of Symbols

1: hydrogen adding device
2: dialyzing water producing device
3: dialysis fluid preparing device
4: concentration adjusting device
5: dialysis machine
6: sensor
7: control device
11: electrolytic cell
100: hemodialysis system
100A: hemodialysis system
100B: hemodialysis system
100C: hemodialysis system
100D: hemodialysis system
100E: hemodialysis system
100F: hemodialysis system

## Claims

1. A hemodialysis system comprising:
a dialyzing water producing device that purifies supplied water and produces dialyzing water;
a dialysis fluid preparing device that adds a medicinal substance to the dialyzing water to prepare a dialysis fluid to be used in hemodialysis treatment;
a dialysis machine that filters patient's blood using the dialysis fluid;
a hydrogen adding device that adds hydrogen to at least one of the water supplied to the dialyzing water producing device, the dialyzing water produced by the dialyzing water producing device, and the dialysis fluid prepared by the dialysis fluid preparing device; and
a concentration adjusting device for adjusting the dissolved hydrogen concentration of the dialysis fluid to be used by the dialysis machine.

2. The hemodialysis system as set forth in claim 1, which comprises
a sensor for measuring the dissolved hydrogen concentration of the dialysis fluid to be used by the dialysis machine; and
a control device that controls the concentration adjusting device based on an output of the sensor.

3. The hemodialysis system as set forth in claim 2, which comprises a plurality of the dialysis machines.

4. The hemodialysis system as set forth in claim 3, which comprises the concentration adjusting device and the sensor corresponding to each of the dialysis machines.

5. The hemodialysis system as set forth in claim 4, wherein
the control device controls the concentration adjusting devices so as to equalize the dissolved hydrogen concentrations of all of the dialysis fluids simultaneously supplied to the plurality of the dialysis machines.

6. The hemodialysis system as set forth in claim 4, wherein
the control device controls the concentration adjusting devices so that the dialysis fluids respectively supplied to the dialysis machines have different dissolved hydrogen concentrations.

7. The hemodialysis system as set forth in claim 1, wherein
the concentration adjusting device includes a plurality of types of concentration adjusting devices.

8. The hemodialysis system as set forth in claim 7, which comprises:
a sensor for measuring the dissolved hydrogen concentration of the dialysis fluid to be used by the dialysis machine; and
a control device that determines a priority order for the operation of each concentration adjusting device based on the output of the sensor and controls each concentration adjusting device based on the priority order.

9. The hemodialysis system as set forth in claim 1, wherein
the hydrogen adding device includes an electrolytic cell that generates hydrogen gas by electrolyzing the supplied water.

10. The hemodialysis system as set forth in claim 9, wherein
the electrolytic cell is also usable as the concentration adjusting device.

11. The hemodialysis system as set forth in claim 1, wherein
at least one of the dialyzing water producing device, the dialysis fluid preparing device and the dialysis machine is provided with a pressure reducing valve for degassing hydrogen gas contained in the dialyzing water or the dialysis fluid, and
the pressure reducing valve is also usable as the concentration adjusting device.

12. The hemodialysis system as set forth in any one of claims 2 to 6 and 8, wherein
at least one of the dialyzing water producing device, the dialysis fluid preparing device and the dialysis machine is provided with an operating device for arbitrarily operating the control device or the concentration adjusting device.
